# EUROPEAN PATENT APPLICATION

(11) **EP 3 926 037 A1**
(43) Date of publication of application: **22.12.2021**
(21) Application number: 20756495.6
(22) Date of filing: 08.01.2020
(51) Int. Cl.: C12M 3/00

(54) **LIQUID THICKNESS CONTROLLING MECHANISM, CULTURE DEVICE, AND LIQUID THICKNESS CONTROLLING METHOD**

(30) Priority: 12.02.2019 JP 2019022448
(71) Applicant: Toyo Seikan Group Holdings, Ltd., Shinagawa-ku Tokyo 141-8627 (JP)
(72) Inventor: KOSEKI Osamu, Yokohama-shi, Kanagawa 240-0062 (JP); TANAKA Satoshi, Yokohama-shi, Kanagawa 240-0062 (JP)
(74) Representative: Liebetanz, Michael
(86) International application number: PCT/JP2020/000210
(87) International publication number: WO 2020/166233

(57) **Abstract**

A liquid thickness of a medium in a culture bag used in cell culture can be controlled. A liquid thickness controlling mechanism that controls the liquid thickness of the medium filled in the culture bag provided with at least one port and formed of a soft packing material includes a detection portion that measures a change caused by a change in the liquid thickness of the medium and a control portion that controls operation of liquid delivery means disposed in a tubular member connecting the culture bag and a medium bag based on detection information inputted from the detection portion so as to control delivery of the medium between the culture bag and the medium bag. Using the liquid thickness controlling mechanism makes it possible to control the liquid thickness of the medium in the culture bag being used.

## Description

### Technical Field

The present invention relates to a cell culture technique, particularly, a technique for efficiently forming spheres and the like.

### Background Art

In recent years, when a large amount of adherent cells such as stem cells including iPS cells and ES cells are cultured, available methods include not only a method in which cells are propagated while being allowed to adhere to a culture vessel, but also a method in which cells are cultured in a three-dimensional state more closely resembling the inside of a living body by allowing the cells to form spheres (spheroids, aggregates) or organoids using a culture vessel provided with a microwell plate or the like to which a material having low adhesiveness to cells is applied.

In mass-culture of the spheres using such a culture vessel, there is a problem in which the spheres jump out of wells (concave parts) and move to other wells, making it difficult to obtain the spheres with a desired size and shape.

For example, when a medium is delivered in a culture bag (a bag-shaped culture vessel) having a large number of wells in a culture portion for exchanging the medium, the spheres easily jump out of the wells and move to other wells. This requires to significantly reduce a flow rate of the medium and makes it very difficult to perform a quick exchange of the medium. Thus, such a culture bag is inadequate for long-term culture requiring frequent medium exchanges.

Further, the spheres easily jump out of the wells upon vibration in such a culture bag, making it difficult to safely transport the culture bag during culturing of the spheres.

### Citation List

### Patent Literature

Patent Literature 1: WO 2016/190312
Patent Literature 2: WO 2016/190314
Patent Literature 3: JP-A-2018-108032

### Summary of Invention

### Technical Problem

Regarding this, the above problem of the sphere relocation can be solved if, in performing the mass-culture of the spheres or the like, a culture bag formed of a soft packing material is used and a liquid thickness of a medium filled in the culture bag can be controlled in a manner such that a size of a gap between an upper surface film and a lower surface film provided with wells of the culture bag is controlled so as to prevent the relocation of the spheres.

However, controlling the liquid thickness of the medium in the culture bag in this manner has not been achieved yet.

Regarding this, controlling the amount of the medium to be delivered to the culture bag has been conventionally performed in order to fill the culture bag with an appropriate amount of the medium according to the number of the cells to be cultured.

For example, the delivery of the medium to the culture bag is controlled by measuring the total weight of the culture bag, a holding plate which holds the culture bag, and the like using a weight sensor and calculating the weight of the medium based on this weight.

Such measurement using the weight sensor is commonly performed by a load cell-type sensor. However, the measurement requires the total weight of the culture bag and the holding plate to be applied on a micro point of the load cell and thus causes a problem of difficulty in fixation and susceptibility to vibration. Further, the weight sensor has poor temperature characteristics, for example, at a low temperature and an installation problem in which, for example, the sensors need to be evenly installed at a plurality of positions. Further, in a case where a piping such a tube is present, there is a problem in which other castings or the like come into contact with the weight sensor via the tube, and, as a result, the tension of the tube is applied to the weight sensor and causes an error in a measurement value.

Further, although such a conventional measurement using the weight sensor can control the delivery amount of the medium based on its weight, it cannot control the liquid thickness of the medium in the culture bag.

As a technique related to the control of the delivery of the medium to the culture bag, a culture device described in Patent Literature 1 can be mentioned.

In this culture device, a distance sensor disposed in a spacer makes it possible to grasp a state in which a distance between a holding plate and the spacer reaches a certain level or more.

However, this culture device neither detects the liquid thickness of the medium in the culture bag nor controls the liquid thickness of the medium.

Further, in a culture device described in Patent Literature 2, a weight detection portion is used to detect the weight of a culture bag and a holding portion of the culture bag. However, again, this culture device neither detects the liquid thickness of the medium in the culture bag nor controls the liquid thickness of the medium.

Further, as a technique that can solve the problem of the sphere relocation, a culture vessel described in Patent Literature 3 can be mentioned. This culture vessel includes a vessel body that includes a bottom portion in which compartments are formed and peripheral wall portions standing up from peripheral parts of the bottom portion, and partition members disposed at positions opposed to the bottom portion, at least a part of each partition member being immersed in a culture liquid in the vessel body. This configuration prevents the relocation of the spheres at the time of transporting the culture vessel or exchanging the medium.

However, such a culture vessel has a problem in which it is difficult to align peripheral wall portions of the compartments and the partition members and adjust gaps between the compartments and the partition members. Further, the warpage of the bottom portion or the partition members makes it further difficult to perform these adjustments. Thus, it is difficult to use this culture vessel as the culture bag formed of the soft packing material.

Regarding this, as a result of intensive studies, the present inventors have succeeded in detecting a liquid thickness of a medium filled in a culture bag formed of a soft packing material and controlling operation of liquid delivery means disposed in a tubular member connecting the culture bag and a medium bag based on the detection information, thereby controlling the liquid thickness of the medium in the culture bag through control of the delivery of the medium. As a result, the present invention has been completed.

According to such a liquid thickness controlling mechanism of the culture bag, it is possible to grasp the liquid thickness of the medium in the culture bag, making it possible to grasp the remaining amount of the medium in the culture bag.

Further, it is possible to grasp a timing in which an upper surface film and a lower surface film of the culture bag come into contact with each other, making it possible to detect abnormality such as a failure or an installation failure of a pump.

Further, it is possible to control the liquid thickness of the medium in the culture bag, making it possible to control a gap between the upper surface film and the lower surface film having a plurality of concave parts of the culture bag to a size that prevents relocation of spheres.

Further, it is also possible to exchange the medium by delivering the medium in the state in which the spheres are prevented from moving between the concave parts in the culture bag.

In view of the foregoing circumstances, an object of the present invention is to provide a liquid thickness controlling mechanism, a culture device, and a liquid thickness controlling method, capable of controlling a liquid thickness of a medium in a culture bag used in cell culture.

### Solution to Problem

In order to achieve the above object, a liquid thickness controlling mechanism of the present invention controls a liquid thickness of a medium filled in a culture bag provided with at least one port and formed of a soft packing material and is configured by including a detection portion that measures a change caused by a change in the liquid thickness of the medium and a control portion that controls operation of liquid delivery means disposed in a tubular member connecting the culture bag and a medium bag based on detection information inputted from the detection portion so as to control delivery of the medium between the culture bag and the medium bag.

Further, a culture device of the present invention controls the liquid thickness of the medium filled in the culture bag provided with at least one port and formed of the soft packing material and is configured by including a frame on which the culture bag is placed, a pressing member that causes the liquid thickness in the culture bag to be uniform by pressing the culture bag against the frame, a top plate portion disposed so as to face the pressing member, a supporting mechanism movably supporting the pressing member in a vertical direction with respect to the top plate portion, a detection portion that measures a change caused by a change in the liquid thickness of the medium, and a control portion that controls the operation of the liquid delivery means disposed in the tubular member connecting the culture bag and the medium bag based on the detection information inputted from the detection portion so as to control the delivery of the medium between the culture bag and the medium bag.

Further, a liquid thickness controlling method of the present invention is a method that controls the liquid thickness of the medium filled in the culture bag provided with at least one port and formed of the soft packing material by using the culture device that includes the frame on which the culture bag is placed, the pressing member that causes the liquid thickness in the culture bag to be uniform by pressing the culture bag against the frame, the top plate portion disposed so as to face the pressing member, the supporting mechanism movably supporting the pressing member in a vertical direction with respect to the top plate portion, the detection portion that measures a change caused by a change in the liquid thickness of the medium, and the control portion that controls the operation of the liquid delivery means disposed in the tubular member connecting the culture bag and the medium bag based on the detection information inputted from the detection portion so as to control the delivery of the medium between the culture bag and the medium bag, in which the control portion stores first information corresponding to a first state in which the liquid thickness of the medium is zero, calculates second information by adding a certain change amount to the first information, and controls the operation of the liquid delivery means based on the second information so as to control the liquid thickness of the medium to a second state corresponding to the second information.

### Advantageous Effects of Invention

According to the present invention, it becomes possible to provide the liquid thickness controlling mechanism, the culture device, and the liquid thickness controlling method, capable of controlling the liquid thickness of the medium in the culture bag used in cell culture.

### Brief Description of Drawings

Fig. 1 is explanatory diagrams illustrating an outline of a culture bag used in a liquid thickness controlling mechanism according to an embodiment of the present invention, (a) is a plan view and (b) is an elevation view.
Fig. 2 is schematic diagrams illustrating parts of culture portions provided with various concave parts of the culture bags used in the liquid thickness controlling mechanism according to the embodiment of the present invention.
Fig. 3 is an explanatory diagram illustrating an outline of a culture device according to the embodiment of the present invention.
Fig. 4 is a plan view illustrating an outline of a culture bag housing portion in the culture device according to the embodiment of the present invention.
Fig. 5 is an elevation view illustrating an outline of the culture bag housing portion in the culture device according to the embodiment of the present invention in a state in which the culture bag is filled with a medium.
Fig. 6 is a left side view illustrating an outline of the culture bag housing portion in the culture device according to the embodiment of the present invention in a state in which the culture bag is filled with the medium.
Fig. 7 is an elevation view illustrating an outline of the culture bag housing portion in the culture device according to the embodiment of the present invention in a state in which the culture bag is not filled with the medium.
Fig. 8 is a left side view illustrating an outline of the culture bag housing portion in the culture device according to the embodiment of the present invention in a state in which the culture bag is not filled with the medium.
Fig. 9 is explanatory diagrams illustrating an outline of a culture bag provided with two ports used in the liquid thickness controlling mechanism according to the embodiment of the present invention, (a) is a plan view and (b) is an elevation view.
Fig. 10 is an explanatory diagram illustrating an outline of a culture device according to the embodiment of the present invention, in which the culture bag provided with two ports is used.

### Description of Embodiments

Hereafter, an embodiment of a liquid thickness controlling mechanism, a culture device, and a liquid thickness controlling method of the present invention will be described in detail. Note that the present invention is not limited to the following specific contents of the embodiment.

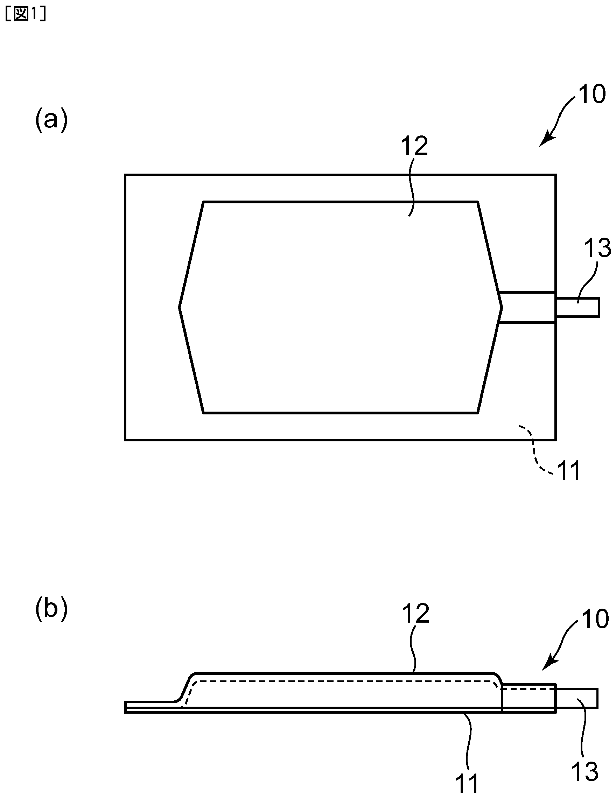

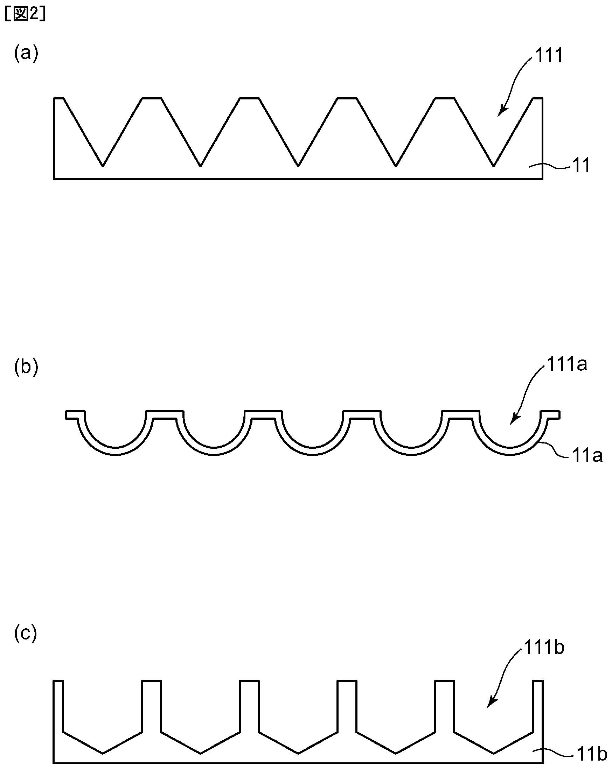

First, a culture bag used in a liquid thickness controlling mechanism according to the present embodiment will be described by referring to Fig. 1 and Fig. 2. Fig. 1 shows explanatory diagrams illustrating an outline of the culture bag used in the liquid thickness controlling mechanism according to the present embodiment with a plan view shown in (a) and an elevation view shown in (b). Fig. 2 shows schematic diagrams illustrating parts of culture portions provided with various concave parts of the culture bags used in the liquid thickness controlling mechanism according to the present embodiment.

A culture bag 10 of the present embodiment formed of a soft packing material can be formed by sticking peripheral parts of a bottom portion-side film 11 (a lower surface film) and a top plate-side film 12 (an upper surface film) together by heat sealing or the like. Further, the culture bag 10 is provided with at least one port 13. This port 13 is connected with a tube, and delivery of the medium such as injection of the medium into the culture bag 10 and discharge of the medium from the culture bag 10 is performed via the port 13 by liquid delivery means disposed in the tube.

Then, a distance (a liquid thickness) of the bottom portion-side film 11 and the top plate-side film 12 is configured to change in accordance with the amount of the medium injected into the culture bag 10.

The surface of the bottom portion-side film 11 in the culture bag 10 is used as a culture portion for culturing cells and spheres. In the culture portion of the bottom portion-side film 11, as shown in Fig. 2, a plurality of concave parts for housing objects to be cultured such as cells and spheres are formed. Forming such concave parts allows the spheres to be suitably formed and cultured. Further, a plurality of the concave parts in the bottom portion-side film 11 can be arranged in, for example, a staggered order or a lattice order.

In Fig. 1, the bottom portion-side film 11 of the culture bag 10 is formed in a plane shape and the top plate-side film 12 is configured to bulge upwards with inflow of the medium. The culture bag 10 configured in this manner can cause the liquid thickness of the medium in the culture bag 10 to be uniform and allow all the concave parts to contact with a mounting surface. Thus, it becomes possible to reduce a difference in cell growth of the objects to be cultured and variability of the number of cells at the time of cell inoculation.

Note that the shape of the culture bag used in the liquid thickness controlling mechanism according to the present embodiment is not limited to such a shape, and the culture bag may be formed by sticking the bottom portion-side film 11 and the top plate-side film 12 having an identical shape by heat sealing or the like.

Further, in Fig. 1, the shape of the culture portion near the port of the culture bag 10 is formed so as to be narrowed to the port, thereby facilitating the flow of the medium to the port. However, the shape of the culture portion is not limited to such a shape and the culture portion may be formed in, for example, a rectangular shape such as rectangle. Further, as described below, the culture bag can be suitable provided with two or more ports.

The shape of the concave parts formed in the culture portion of the bottom portion-side film 11 can be a conical shape such as a circular cone or a square cone as shown in Fig. 2(a), or a hemispherical shape or a round shape as shown in Fig. 2(b).

Further, it is also preferable that the concave parts formed in the culture portion of the bottom portion-side film 11 have a shape in which at least parts of side walls are substantially vertically formed as shown in Fig. 2(c). In these concave parts, the length of the substantially vertical portions of the side walls in a vertical direction is longer than a half of the maximum diameter of the objects to be cultured. Further, the regions of the concave parts in which the side walls are substantially vertically can be formed in, for example, a cylindrical shape, a quadrangular prism shape, or the like.

The concave parts in the culture bag 10 having such a shape can prevent the objects to be cultured from jumping out of the concave parts and move to other concave parts even when the medium is delivered in the culture bag 10 from the port 13 at a flow rate of 1 ml/min or more.

In a case where the objects to be cultured are spheres, the depth of the concave parts is preferably from 50 to 500 µm. When the depth of the concave parts is more than 500 µm, it is sometimes difficult to sufficiently exchange the medium in the concave parts by delivering the medium to the culture bag 10, and it becomes more difficult to process the culture vessel provided with the concave parts having a depth of more than 500 µm. Further, the above range is preferable because the size of the smaller spheres is about from 50 µm to 100 µm and the size of the larger spheres is about from 200 µm to 300 µm.

The shape of the bottom portions in the concave parts is not particularly limited. In a case where the objects to be cultured are the spheres, the shape is preferably a cone shape, a hemispherical shape, or a round shape for suitably forming the spheres by facilitating aggregation of single cells.

In a case where the objects to be cultured are single cells, the depth (a) of the concave parts is preferably from 5 to 50 µm. This is because the size of the single cells is about from 6 µm to 15 µm, mostly about 10 µm.

The shape of opening portions of the concave parts is not particularly limited. It can be a circular shape or a rectangular shape such as square. Further, the width of the opening portions of the concave parts can be appropriately set according to the size of the objects to be cultured.

For example, in a case where the objects to be cultured are the spheres, the lower limit of the diameter of the circle or the inscribed circle of the opening portions of the concave parts may be set to 60 µm or more, 70 µm or more, 80 µm or more, 90 µm or more, 100 µm or more, 110 µm or more, 120 µm or more, 150 µm or more, or the like. Further, the upper limit of the diameter of the circle or the inscribed circle of the opening portions of the concave parts may be set to 1 mm or less, 900 µm or less, 800 µm or less, 700 µm or less, 500 µm or less, or the like.

Further, in a case where the objects to be cultured are the single cells, the lower limit of the diameter of the circle or the inscribed circle of the opening portions of the concave parts may be set to 5 µm or more, 6 µm or more, 8 µm or more, or the like. Further, the upper limit of the diameter of the circle or the inscribed circle of the opening portions of the concave parts may be set to 50 µm or less, 40 µm or less, 30 µm or less, or the like.

Aa a material of the culture bag 10 used in the liquid thickness controlling mechanism according to the present embodiment, a polyolefin resin such as polyethylene or polypropylene or the like can be suitably used. For example, polyethylene, a copolymer of ethylene and α-olefin, a copolymer of ethylene and vinyl acetate, an ionomer using a copolymer of ethylene and acrylic acid or methacrylic acid and a metal ion, or the like can be mentioned. Further, polyolefin, a styrene-based elastomer, a polyester-based thermoplastic elastomer, a silicone-based thermoplastic elastomer, a silicone resin, or the like can be also used. Further, silicone rubber, a soft vinyl chloride resin, a polybutadiene resin, an ethylene-vinyl acetate copolymer, a chlorinated polyethylene resin, a polyurethane-based thermoplastic elastomer, a polyester-based thermoplastic elastomer, a silicone-based thermoplastic elastomer, a styrene-based elastomer such as SBS (styrene-butadiene-styrene), SIS (styrene-isoprene-styrene), SEBS (styrene-ethylene-butylene-styrene), or SEPS (styrene-ethylene-propylene-styrene), a polyolefin resin, a fluoro resin, or the like may be used.

Further, the culture portion of the bottom portion-side film 11 is preferably subjected to low adhesion surface treatment coating to prevent adhesion of the spheres and the single cells. Specifically, a cell adhesion inhibitor (a low cell-adhesion treating agent) is preferably applied.

As the cell adhesion inhibitor, a phospholipid polymer, a polyvinyl alcohol derivative, a phospholipid-polymer complex, polyhydroxyethylmethacrylate, a polyvinyl alcohol, agarose, chitosan, polyethylene glycol, albumin, or the like can be used. Further, a combination thereof may be used.

Further, as a material of the port 13 in the culture bag 10, the port 13 may be molded using, for example, polyethylene, polypropylene, vinyl chloride, a polystyrene-based elastomer, a thermoplastic resin such as FEP, or the like.

Next, the liquid thickness controlling mechanism according to the present embodiment will be described. The liquid thickness controlling mechanism of the present embodiment controls the liquid thickness of the medium filled in the culture bag provided with at least one port and formed of the soft packing material and is characterized by including a detection portion for measuring a change caused by a change in the liquid thickness of the medium and a control portion for controlling operation of the liquid delivery means disposed in the tubular member connecting the culture bag and the medium bag based on detection information inputted from the detection portion, thereby controlling the delivery of the medium between the culture bag and the medium bag.

As the change caused by a change in the liquid thickness of the medium, displacement of a position of a member can be used, and, as the detection portion, a length measurement sensor can be suitably used.

Further, the control portion can calculate the liquid thickness of the medium based on the detection information and control the operation of the liquid delivery means based on this liquid thickness.

As the length measurement sensor, various sensors such as an electromagnetic induction type sensor, a laser type sensor, an ultrasonic type sensor, and a magnetic type sensor can be used. Further, between analog (linear) type and switch type sensors, an analog type sensor performing continuous measurement is preferably used.

Further, in a case where an electromagnetic induction type length measurement sensor or the like is used, a metal member is preferably installed at the position to be measured. This can improve sensitivity of the sensor. Note that, in a case where a laser type or ultrasonic type length measurement sensor is used, the measurement can be performed without installing the metal member at the position to be measured.

Specifically, in a case where an analog proximity sensor (an electromagnetic induction type, a measurement distance of from 1 to 6 mm, MDA-C5, manufactured by SENSATEC Co., Ltd.) is used as the length measurement sensor, an output voltage (or an output current) is substantially proportional to a measurement distance, making it possible to detect a 0.01 mm incremental change in the liquid thickness (the liquid thickness of the medium in the culture bag) per 0.01 V output voltage in a range of the measurement distance.

For example, in a case where the culture bag in which the area of the culture portion is 50 cm² is used, the liquid volume of the medium corresponding to the liquid thickness of 0.01 mm is 0.05 ml. In this case, when the delivery rate is set to 2 ml/min, the medium can be delivered in 1.5 seconds and the liquid thickness can be increased or decreased by 0.01 mm. Further, when the delivery rate is set to 0.5 ml/min, the medium can be delivered in 6 seconds and the liquid thickness can be increased or decreased by 0.01 mm.

Further, when an analog distance sensor (an electromagnetic induction type, a measurement distance of from 0 to 12 mm, PE2-LA10D, manufactured by Fuji Electric FA Components & Systems Co., Ltd.) is used as the length measurement sensor, the liquid thickness can be controlled to a larger extent, for example, up to about 8 mm.

When the liquid thickness is directly measured using the length measurement sensor, for example, the length measurement sensor is disposed on the frame on which the culture bag is placed and the metal member is disposed on the pressing member, so that a distance to the metal member can be measured by the length measurement sensor.

In this case, the liquid thickness can be obtained by multiplying the output voltage of the length measurement sensor by a predetermined coefficient and performing correction by subtracting the thickness of the films and the like.

Further, as described below, the length measurement sensor is disposed on the top plate portion supporting the pressing member and the metal member is disposed on the pressing member, so that a distance to the metal member can be measured by the length measurement sensor.

In this case, the liquid thickness can be obtained by multiplying the output voltage of the length measurement sensor by a predetermined coefficient to calculate the distance from the length measurement sensor to the metal member and subtracting the above calculated distance, the thickness of the metal member, the height of the pressing member, and the thickness of the films from the distance from the upper surface of the frame on which the culture bag is placed to the lower surface of the length measurement sensor.

Further, as the change caused by a change in the liquid thickness of the medium, a change in the weight of the culture bag housing portion in which the culture bag is placed is used, and, as the detection portion, a weight sensor can be used. Then, the control portion can calculate the liquid thickness of the medium based on the change in the weight and control the operation of the liquid delivery means based on this liquid thickness.

That is, the liquid thickness is calculated by subtracting the weight at the time when the liquid thickness of the culture bag is zero from the weight at the time of measurement, converting the obtained value to the volume, and then dividing the converted volume by the area of the culture portion. Then, the operation of the liquid delivery means can be controlled based on this liquid thickness.

According to such a liquid thickness controlling mechanism of the present embodiment, it is possible to grasp the liquid thickness of the medium in the culture bag, making it possible to grasp the remaining amount of the medium in the culture bag.

Further, it is possible to grasp a timing in which the upper surface film and the lower surface film of the culture bag come into contact with each other. This makes it possible to prevent a situation in which there is no medium left in the culture bag. Further, it becomes possible to detect a delivery error due to a failure or inappropriate installation of the pump.

Further, the liquid thickness of the medium in the culture bag can be controlled by feedback of the detection value of the liquid thickness of the medium to the control portion, thus it becomes possible to control the gap between the upper surface film and the lower surface film having a plurality of the concave parts of the culture bag to a size that prevents the relocation of the spheres (a gap of about 50 µm).

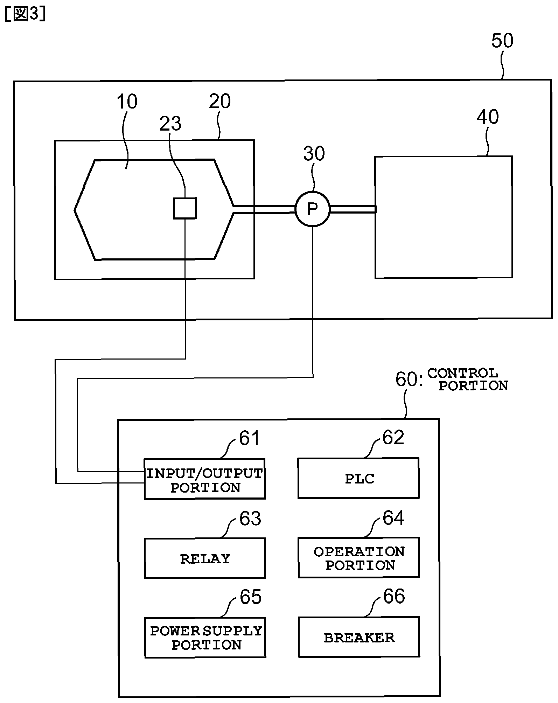

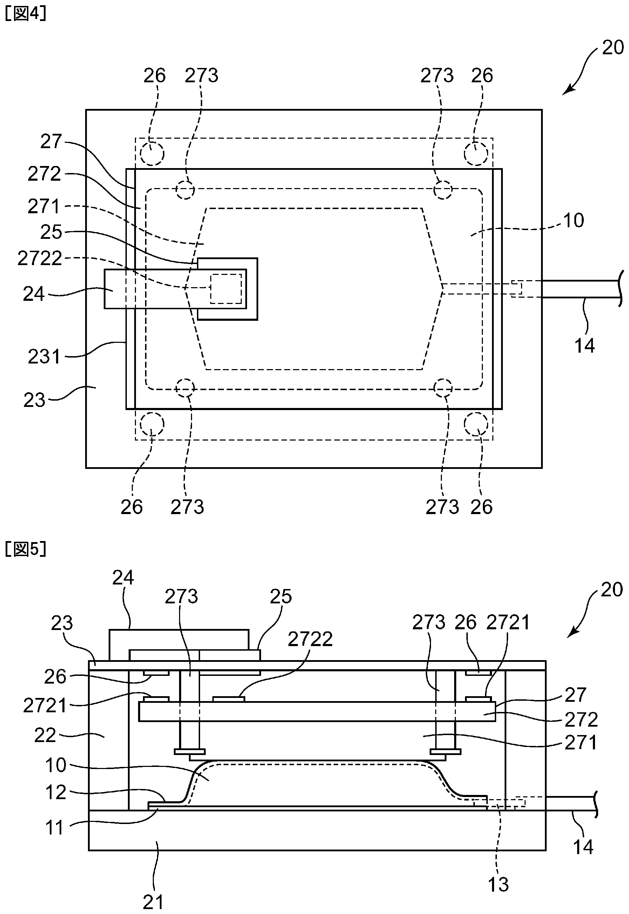

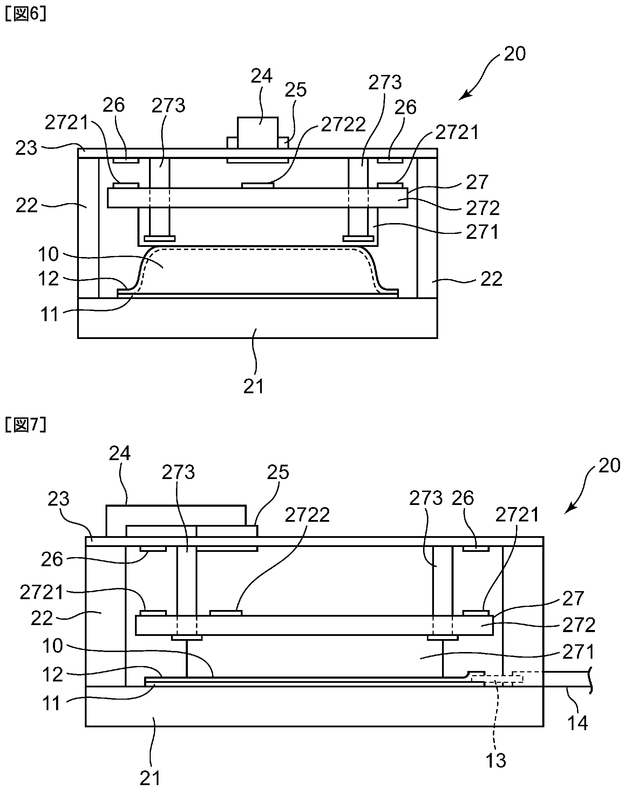

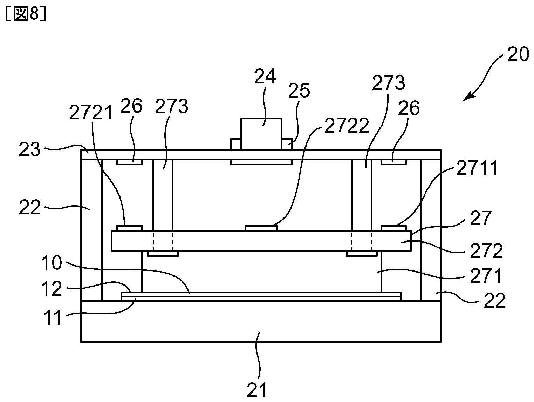

Next, the culture device and the liquid thickness controlling method according to the present embodiment will be described with reference to Fig. 3 to Fig. 8. Fig. 3 shows an explanatory diagram illustrating an outline of the culture device according to the present embodiment. Fig. 4 shows a plan view illustrating an outline of the culture bag housing portion in the culture device according to the present embodiment. Fig. 5 shows an elevation view illustrating an outline of the culture bag housing portion in a state in which the culture bag is filled with the medium, while Fig. 6 shows a left side view illustrating an outline of the culture bag housing portion in a state in which the culture bag is filled with the medium. Fig. 7 shows an elevation view illustrating an outline of the culture bag housing portion in a state in which the culture bag is not filled with the medium, while Fig. 8 shows a left side view illustrating an outline of the culture bag housing portion in a state in which the culture bag is not filled with the medium. Note that, in these drawings, closing means such as a pinch valve for closing a flow passage of the tube is omitted.

The culture device of the present embodiment controls the liquid thickness of the medium filled in the culture bag provided with at least one port and formed of the soft packing material and is characterized by including a frame on which the culture bag is placed, a pressing member for causing the liquid thickness in the culture bag to be uniform by pressing the culture bag against the frame, a top plate portion disposed so as to face the pressing member, a supporting mechanism movably supporting the pressing member in a vertical direction with respect to the top plate portion, a detection portion for measuring a change caused by a change in the liquid thickness of the medium, and a control portion for controlling operation of liquid delivery means disposed in a tubular member connecting the culture bag and a medium bag based on detection information inputted from the detection portion, thereby controlling the delivery of the medium between the culture bag and the medium bag.

Specifically, as shown in Fig. 3, the culture device includes a culture bag housing portion 20, a culture bag 10 placed in the culture bag housing portion 20, a medium bag 40, liquid delivery means 30 disposed in a tube connecting the culture bag 10 and the medium bag 40, and a control portion 60 connected to a length measurement sensor 25 and the liquid delivery means 30. All components other than the control portion 60 are disposed inside an incubator 50 during culturing.

In the culture bag housing portion 20, top plate supporting portions 22 are vertically arranged at four corners of a frame 21, and a top plate portion 23 is fixed to the top plate supporting portions 22. Further, guide pins 273 are provided in peripheral parts of the top plate portion 23 so as to pass through guide holes disposed in attachment parts 272 of a pressing member 27. The pressing member 27 is provided movably in a vertical direction along the guide pins 273.

Further, a top plate opening portion 231 is provided in the top plate portion 23, and an inside of the culture device 20 can be viewed through the top plate opening portion 231.

Further, permanent magnets 26 are provided at four corners of the top plate portion 23 as biasing means for biasing the pressing member 27 downward, and permanent magnets 2721 are provided on the attachment parts 272 at positions corresponding to the permanent magnets 26 as biasing means. These permanent magnets are arranged with like poles facing to each other, and a repulsion acting between the permanent magnets 26 and the permanent magnets 2721 allows a pressing portion 271 of the pressing member 27 to move in a vertical direction in a state in which the pressing portion 271 vertically presses the top plate-side film 12 of the culture bag 10.

Note that biasing means 73 is not limited to the permanent magnet. Further, the pressing member 27 may be allowed to move up and down with respect to the top plate-side film 12 by its own weight without using the biasing means 73.

Further, the pressing member 27 is sized so that the bottom surface of the pressing portion 271 is arranged inside the culture portion of the culture bag 10.

Pressing the culture bag 10 by the pressing member 27 in this manner can prevent the occurrence of unevenness of the top plate-side film 12. As a result, the top plate-side film 12 is held parallel to a mounting surface of the frame 21 when the medium is injected and discharged, causing the liquid thickness of the medium in the culture bag 10 to be uniform.

The pressing member 27 may be formed of, for example, a synthetic resin such as polycarbonate, or glass. Further, a part or the whole of the pressing member 27 is preferably transparent in order to confirm the progress of culturing, a state of the objects to be cultured, and the like.

When the culture bag 10 is filled with the medium, as shown in Fig. 5 and Fig. 6, the pressing member 27 is in a state of being pushed up by the culture bag 10.

On the other hand, when the medium is discharged from the culture bag 10 via a tube 14 connected to the port 13 of the culture bag 10 by the liquid delivery means 30 disposed in the tube 14, the liquid thickness of the medium in the culture bag 10 is reduced accordingly, thereby causing the pressing member 27 to move downward.

Then, when the medium is completely discharged from the culture bag 10, as shown in Fig. 7 and Fig. 8, the culture bag 10 is pressed by the pressing member 27 to a state in which the upper surface film and the lower surface film contact each other (a state in which a plurality of concave parts formed in the bottom portion-side film 11 are closed by the plate-side film 12). At this moment, the liquid thickness of the medium in the culture bag 10 becomes zero.

A sensor supporting portion 24 is fixed to a peripheral part of the top plate portion 23 and a length measurement sensor 25 is attached to a tip part of the sensor supporting portion 24. Further, a metal member 2722 is attached to the attachment part 272 of the pressing member 27 so as to face the length measurement sensor 25. Note that the arrangement of the sensor supporting portion 24 and the length measurement sensor 25 is not limited thereto, and they can be attached to any position of the top plate portion 23 within a range where the distance to the metal member 2722 can be measured.

Then, the distance to the metal member 2722 is measured by the length measurement sensor 25 and the liquid thickness of the medium in the culture bag 10 is calculated, so that the liquid thickness can be detected.

For example, the liquid thickness can be obtained by multiplying the output voltage of the length measurement sensor 25 by a predetermined coefficient to calculate the distance from the length measurement sensor 25 to the metal member 2722 and subtracting the above calculated distance, the thickness of the metal member 2722 , the height of the pressing member, and the thickness of the upper surface film and the lower surface film from the distance from the upper surface of the frame 21 to the lower surface of the length measurement sensor 25.

The liquid delivery means 30 is disposed in the tube connecting the culture bag 10 and the medium bag 40. As the liquid delivery means 30, for example, a pump capable of performing liquid delivery at a low rate and with high accuracy, such as a peristaltic pump or a syringe pump, is preferably used. Note that the liquid delivery means 30 is not limited to these pumps, and a hydraulic head system (own weight), a pressurizing system, or the like can be used.

Operating such liquid delivery means 30 allows the medium to be filled in the culture bag 10 from the medium bag 40 and to be discharged from the culture bag 10 to the medium bag 40.

The operation of the liquid delivery means 30 is controlled by the control portion 60 based on detection information from the length measurement sensor 25. In this operation, the control portion 60 calculates the liquid thickness of the medium in the culture bag 10 based on the output voltage (or the output current) inputted (sent) from the length measurement sensor 25 and controls the start and stop of the operation, a rotation speed, and the like of the liquid delivery means 30 based on the liquid thickness, thereby making it possible to control the liquid thickness to a desired size.

The medium bag 40 can be used as a medium supplying bag for supplying the medium or a medium discharging bag for discharging the medium.

The incubator 50 housing the culture bag 10, the culture bag housing portion 20, the liquid delivery means 30, and the medium bag 40 maintains a constant temperature and humidity to keep a culture environment of cells and spheres in the culture bag 10.

The control portion 60 (a control unit) is not particularly limited as long as it can control the operation of the liquid delivery means 30 based on the detection information from the length measurement sensor 25. It can be configured by including an input/output portion 61 (an input/output unit), a PLC (programmable logic controller) 62, a relay 63, an operation portion 64 (a touch panel, etc.), a power supply portion 65 (a stabilized power supply, etc.), and a breaker 66. Further, such a configuration may be achieved by using a microcomputer or a computer.

A desired control content is programmed and stored in the PLC 62 in advance, and the operation of the liquid delivery means 30 can be controlled based on this.

For example, the PLC 62 in the control portion 60 stores first information corresponding to a first state in which the liquid thickness of the medium is zero, calculates second information by adding a certain change amount to the first information, and control the operation of the liquid delivery means 30 based on the second information, so that the liquid thickness of the medium can be controlled to a second state corresponding to the second information. Note that, as the first information and the second information, a voltage, a current, a transistor output, the liquid thickness of the medium, or the like can be used.

Specifically, the medium in the culture bag 10 is discharged, the output voltage corresponding to the state in which the liquid thickness is zero is stored, and then driving of the liquid delivery means 30 such as a pump is controlled based on the detection information from the length measurement sensor 25, so that the medium can be supplied to the culture bag 10 until the medium in the culture bag 10 reaches the desired liquid thickness.

The culture device and the liquid thickness controlling method according to the present embodiment are not limited to the above configurations and methods. Other configurations and methods can be employed as long as the liquid thickness controlling mechanism according to the present embodiment can be implemented.

For example, the culture device can be configured such that the length measurement sensor is disposed on the frame on which the culture bag is placed, and the metal member is disposed on the pressing member. Further, the culture device can be configured such that the weight of the culture bag 10 including the medium is detected using the weight sensor and outputted to the control portion 60, and the control portion 60 calculate the liquid thickness of the medium based on the weight of the medium and controls the operation of the liquid delivery means 30.

Further, it is also preferable that, as the culture bag used in the liquid thickness controlling mechanism according to the present embodiment, a culture bag provided with two ports is used, first liquid delivery means is disposed in a tubular member connecting a first port and a first bag, second liquid delivery means is disposed in a tubular member connecting a second port and a second bag, and the control portion controls the operation of the first liquid delivery means and the second liquid delivery means based on the detection information inputted from the detection portion.

That is, it is also preferable that the culture device and the liquid thickness controlling method according to the present embodiment are achieved by disposing a pump or the like in the tube connected to each of two ports.

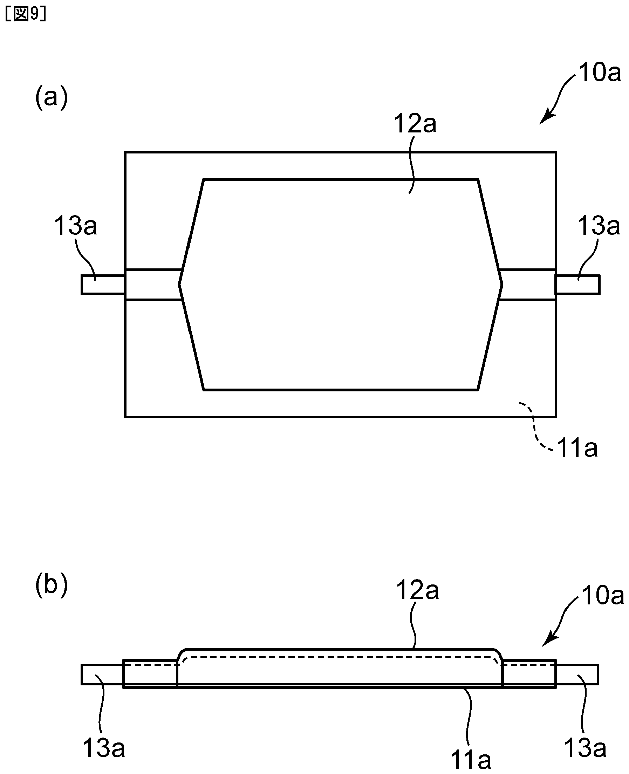

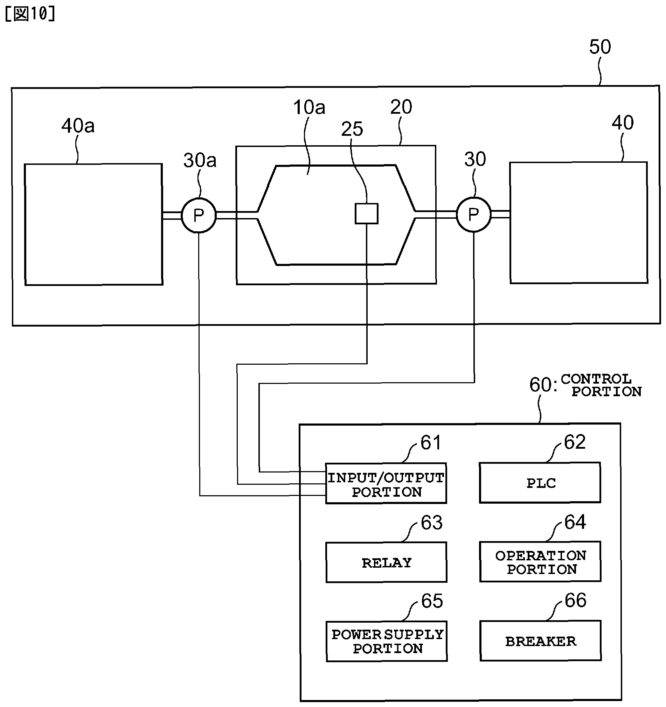

Specifically, a culture bag 10a provided with two ports 13a as shown in Fig. 9 and Fig. 10 can be suitably used.

In these drawings, one port 13a of the culture bag 10a is connected to the medium bag 40 via the tube, and the liquid delivery means 30 is disposed in this tube. In this configuration, the medium bag 40 can be used as the medium supplying bag or the like. Further, the other port 13a of the culture bag 10a is connected to the discharging bag 40a via the tube, and the liquid delivery means 30a is disposed in this tube. Note that the bag connected to each port via the tube may be a bag for other uses.

The culture device according to the present embodiment having such a configuration makes it possible to control the operation of the liquid delivery means 30 and the liquid delivery means 30a based on the detection information from the length measurement sensor 25.

In this configuration, for example, the medium can be exchanged by delivering the medium from the medium bag 40 to the culture bag 10a and delivering the medium from the culture bag 10a to the discharging bag 40a while the liquid thickness of the medium in the culture bag 10a is maintained in a size that prevents the relocation of the spheres (a gap of about 50 µm). Further, in this configuration, a delivery rate of the medium can be also controlled by controlling the operation of the liquid delivery means 30 and the liquid delivery means 30a.

Further, the culture bag used in the liquid thickness controlling mechanism according to the present embodiment preferably has a configuration such that top plate projections are provided on the top plate-side film 12 inside the culture bag 10, the width of the top plate projections is made smaller than the width of the opening portions of the concave parts formed on the bottom portion-side film 11, and the height of the top plate projections is made smaller than the minimum diameter of the objects to be cultured, so that, when the top plate projections come into contact with parts of the upper end surface of the culture portion, gaps are formed between the upper end surface and the lower surface of the top plate-side film 12.

In the culture bag used in the liquid thickness controlling mechanism according to the present embodiment having such a configuration, when the top plate projections come into contact with the parts of the upper end surface, the gaps smaller than the size of the objects to be cultured can be formed between the upper end surface and the top plate portion. This allows a liquid object such as the medium to pass through the gaps while preventing the relocation of the objects to be cultured such as the spheres.

In a case where the objects to be cultured are the spheres, the culture device according to the present embodiment can be used in a step of forming the spheres, a step of culturing and propagating cells by keeping a sphere state, a step of inducing differentiation in a sphere state, and other steps.

Further, it can be used in a method in which the spheres are formed using differentiated cells that have been obtained by subjecting iPS cells, ES cells, or other stem cells to a differentiation-inducing step, a method in which the spheres are formed using cells that have been differentiated, cryopreserved, and then thawed, and other methods.

As described above, according to the liquid thickness controlling mechanism, the culture device, and the liquid thickness controlling method according to the embodiment of the present invention, it is possible to grasp the liquid thickness of the medium in the culture bag, making it possible to grasp the remaining amount of the medium in the culture bag.

Further, it is possible to grasp a timing in which the upper surface film and the lower surface film of the culture bag come into contact with each other. This makes it possible to prevent a situation in which there is no medium left in the culture bag. Further, it becomes possible to detect a delivery error due to a failure or inappropriate installation of the pump.

Further, the liquid thickness of the medium in the culture bag can be controlled by the feedback of the detection value of the liquid thickness of the medium to the control portion, thus it becomes possible to control the gap between the upper surface film and the lower surface film having a plurality of concave parts of the culture bag to a size that prevents the relocation of the spheres.

Further, when the culture bag provided with two ports are used and the pump is disposed in each of the tubes connected to two ports, for example, it becomes possible to exchange the medium by delivering the medium from the medium bag to the culture bag and delivering the medium from the culture bag to the discharging bag in a state in which the liquid thickness of the medium in the culture bag is maintained in a size that prevents the relocation of the spheres.

The present invention is not limited to the above embodiment, and it is needless to say that various modifications can be made within the scope of the present invention. As an example of appropriate modifications, a configuration is made such that the culture bag is provided with three or more ports, the pump is disposed in each of the tubes connected to these ports, and the pumps are controlled based on information from a detector, or the position of the pressing member or the length measurement sensor in the culture device is changed.

### Industrial Applicability

The present invention can be suitably used in a case where the spheres or the like with a uniform size are efficiently mass produced, and the like.

The documents described in this specification and the Japanese application specification claiming priority under the Paris Convention are incorporated herein by reference in its entirety.

### Reference Signs List

- 10: Culture bag
- 11, 11a, 11b: Bottom portion-side film
- 111, 111a, 111b: Concave part
- 12: Top plate-side film
- 13: Port
- 14: Tube (tubular member)
- 20: Culture bag housing portion
- 21: Frame
- 22: Top plate supporting portion
- 23: Top plate portion
- 231: Top plate opening portion
- 24: Sensor supporting portion
- 25: Length measurement sensor
- 26: Permanent magnet
- 27: Pressing member
- 271: Pressing portion
- 272: Attachment part
- 2721: Permanent magnet
- 2722: Metal member
- 273: Guide pin
- 30: Liquid delivery means
- 40: Medium bag
- 50: Incubator
- 60: Control portion
- 61: Input/output portion
- 62: PLC (programmable logic controller)
- 63: Relay
- 64: Operation portion
- 65: Power supply portion
- 66: Breaker

## Claims

1. A liquid thickness controlling mechanism for controlling a liquid thickness of a medium filled in a culture bag provided with at least one port and formed of a soft packing material, the liquid thickness controlling mechanism comprising:
a detection portion that measures a change caused by a change in the liquid thickness of the medium; and
a control portion that controls operation of liquid delivery means disposed in a tubular member connecting the culture bag and a medium bag based on detection information inputted from the detection portion so as to control delivery of the medium between the culture bag and the medium bag.

2. The liquid thickness controlling mechanism according to claim 1, wherein
the change caused by the change in the liquid thickness of the medium is displacement of a position of a member, and the detection portion is a length measurement sensor.

3. The liquid thickness controlling mechanism according to claim 1, wherein
the change caused by the change in the liquid thickness of the medium is a change in weight of a housing portion in which the culture bag is placed, and the detection portion is a weight sensor.

4. The liquid thickness controlling mechanism according to any of claims 1 to 3, wherein
the control portion calculates the liquid thickness of the medium and controls the operation of the liquid delivery means based on this liquid thickness.

5. A culture device that controls a liquid thickness of a medium filled in a culture bag provided with at least one port and formed of a soft packing material, the culture device comprising:
a frame on which the culture bag is placed;
a pressing member that causes the liquid thickness in the culture bag to be uniform by pressing the culture bag against the frame;
a top plate portion disposed so as to face the pressing member;
a supporting mechanism movably supporting the pressing member in a vertical direction with respect to the top plate portion;
a detection portion that measures a change caused by a change in the liquid thickness of the medium; and
a control portion that controls operation of liquid delivery means disposed in a tubular member connecting the culture bag and a medium bag based on detection information inputted from the detection portion so as to control delivery of the medium between the culture bag and the medium bag.

6. The culture device according to claim 5, wherein
the change caused by the change in the liquid thickness of the medium is displacement of a position of a member, and the detection portion is a length measurement sensor.

7. The culture device according to claim 5 or 6, wherein
the control portion calculates the liquid thickness of the medium and controls the operation of the liquid delivery means based on this liquid thickness.

8. The culture device according to any of claims 5 to 7, wherein
the length measurement sensor is disposed on the top plate portion and measures a distance to a metal member disposed on the pressing member.

9. The culture device according to any of claims 5 to 7, wherein
the length measurement sensor is disposed on the frame and measures the distance to the metal member disposed on the pressing member.

10. The culture device according to any of claims 5 to 9, wherein
the pressing member includes a pressing portion having a horizontal surface in a shape, the pressing portion presses the culture bag, the shape is the same as a shape of a horizontal surface of a culture portion of the culture bag, and the pressing portion presses only an inside of a region of the culture portion.

11. The culture device according to any of claims 5 to 10, wherein
a plurality of concave parts for housing cells or spheres are formed in the culture portion of the culture bag.

12. A liquid thickness controlling method for controlling a liquid thickness of a medium filled in a culture bag provided with at least one port and formed of a soft packing material, wherein:
the liquid thickness controlling method uses a culture device including:
a frame on which the culture bag is placed;
a pressing member that causes the liquid thickness in the culture bag to be uniform by pressing the culture bag against the frame;
a top plate portion disposed so as to face the pressing member;
a supporting mechanism movably supporting the pressing member in a vertical direction with respect to the top plate portion;
a detection portion that measures a change caused by a change in the liquid thickness of the medium; and
a control portion that controls operation of liquid delivery means disposed in a tubular member connecting the culture bag and a medium bag based on detection information inputted from the detection portion so as to control delivery of the medium between the culture bag and the medium bag; and
the control portion stores first information corresponding to a first state in which the liquid thickness of the medium is zero, calculates second information by adding a certain change amount to the first information, and controls the operation of the liquid delivery means based on the second information so as to control the liquid thickness of the medium to a second state corresponding to the second information.

13. The liquid thickness controlling method according to claim 12, wherein
the change caused by the change in the liquid thickness of the medium is displacement of a position of a member, and the detection portion is a length measurement sensor.

14. The liquid thickness controlling method according to claim 12 or 13, wherein
the first information and the second information are any of a voltage, a current, a transistor output, or the liquid thickness of the medium.

15. The liquid thickness controlling method according to any of claims 12 to 14, wherein
the culture bag provided with two ports is used, first liquid delivery means is disposed in a tubular member connecting a first port and a first bag, second liquid delivery means is disposed in a tubular member connecting a second port and a second bag, and the control portion controls operation of the first liquid delivery means and the second liquid delivery means based on the detection information inputted from the detection portion.
